(19) Europäisches Patentamt
European Patent Office
Office européen des brevets

(11) Publication number: **0 217 814 B2**

(12) **NEW EUROPEAN PATENT SPECIFICATION**

(45) Date of publication of the new patent specification: **21.12.94**

(51) Int. Cl.[5]: **C12P 21/06**, C12N 15/18

(21) Application number: **86901361.5**

(22) Date of filing: **06.02.86**

(86) International application number: **PCT/DK86/00014**

(87) International publication number: **WO 86/04609 (14.08.86 86/18)**

# (54) A PROCESS FOR PRODUCING HUMAN GROWTH HORMONE.

(30) Priority: **07.02.85 DK 556/85**

(43) Date of publication of application: **15.04.87 Bulletin 87/16**

(45) Publication of the grant of the patent: **16.05.90 Bulletin 90/20**

(45) Mention of the opposition decision: **21.12.94 Bulletin 94/51**

(84) Designated Contracting States: **AT BE CH DE FR GB IT LI LU NL SE**

(56) References cited:
**EP-A- 0 020 290**
**EP-A- 0 035 384**
**EP-A- 0 089 626**
**EP-A- 0 127 305**
**WO-A-84/02351**
**US-A- 4 543 329**

**Chemical Abstracts, vol. 77, 1972, Abstract no. 2289q, Calluhan et al, Fed. Proc. Fed. Amer, Coc. Exp. Biol. 1972 31(3), 1105-13 (Eng).**

(73) Proprietor: **NOVO NORDISK A/S**
**Novo Allé**
**DK-2880 Bagsvaerd (DK)**

(72) Inventor: **ANDERSEN, Henrik, Dalboge**
**Ordrupvej 112 B**
**DK-2920 Charlottenlund (DK)**
Inventor: **PEDERSEN, John**
**Kajerodvaenge 35**
**DK-3460 Birkero /d (DK)**
Inventor: **CHRISTENSEN, Thorkild**
**Bellisvej 55**
**DK-3450 Allerod (DK)**
Inventor: **HANSEN, Jorli, Winnie**
**Kloverhoj 20**
**DK-2600 Glostrup (DK)**
Inventor: **JESSEN, Torben, Ehlern**
**Kalundborgvej 216**
**DK-4300 Holbaek (DK)**

(74) Representative: **Christiansen, Ejvind et al**
**HOFMAN-BANG & BOUTARD A/S**
**Adelgade 15**
**DK-1304 Copenhagen K (DK)**

**EP 0 217 814 B2**

**Chemical Abstracts, vol. 76, 1972, Abstract no. 83215s, Lindley et al. Biochem J. 1972, 126(3), 683-5(Eng).**

**Bio.Technology; February 1984, 161-165**

**Blumberg et al.: 1984, Preparation of Authentic Human Growth Hormone by Genetic Engineering and Subsequent "Processing", Biotech. '84, USA, Online Publications, Pinner UK, pp. A285-295**

## Description

The present invention concerns a process for producing authentic human growth hormone (hGH) from amino terminal extended hGH using Dipeptidyl Aminopeptidase I (DAP I) (E.C. 3.4. 14.1).

It is known from the US Patent Specification 4 342 832 to produce biosynthetic hGH by fermentation of a recombinant host cell, in particular Escherichia coli, which codes for hGH with associated methionine. However, this known process results in hGH whose N-terminus has attached to it the amino acid methionine which is not present in authentic hGH.

Owing to the risk of antigenic reactions and other side effects in the use of a growth hormone which is not quite identical with hGH, there is a great need for a process which enables production of biosynthetic hGH with a correct amino acid sequence. A solution to this problem has been proposed in EP-A-127 305, which concerns a process for producing hGH from pre-hGH in a recombinant prokaryotic microorganism, such as Pseudomonas aeruginosa or E. coli.

The use of Pseudomonas aeruginosa for the production of hGH without methionine for therapeutic use, however, is vitiated by the risk that this bacterium and many other Pseodomonas bacteria, which are potentially pathogenic, synthetize toxic toxines which are difficult to remove by purification of hGH.

The expression of pre-hGH followed by proteolytic cleavage to obtain the authentic hGH in an E. coli (which is not pathogenic) is indicated in EP-A-127 305, but it is not documented in that specification that the proteolytic cleavage unambiguously leads to the formation of authentic hGH, i.e. with a correct amino acid sequence.

As mentioned above, risks may be involved in using Met-hGH. Though methods have been proposed for enzymatic cleavage of the methionine group by means of aminopeptidases, the problem would not be solved by this because the known enzymatic processes of this type do not lead to a 100% conversion. A mixture of hGH and Met-hGH would occur, which cannot be separated completely by conventional preparative purification processes.

In EP-A-89626 a method of producing specific polypeptides is described, wherein a fusion product is expressed consisting of the polypeptide and a polymer of charged amino acids. Following a separation of the fusion product from contaminants based upon the properties of said polymer, it is removed using an exopeptidase. No specific guidance as to the formation of hGH is given.

In EP-A-35384 the use of special purification linkers with a specific cleavage site for endopeptidases is suggested. The purification linker may contain charged amino acids. No specific guidance as to the formation of hGH is given.

In WO84/02351 a process for producing i.a. authentic hGH is suggested, wherein a biosynthetically formed N-terminal extended hGH is digested with an aminopeptidase, preferably leucine aminopeptidase (LAP) in order to cleave the extension. The extension consisted of arbitrarily selected amino acids which were removed by stepwise cleavage. However, this process did not work in practice with pure LAP.

The present invention is based on the surprising recognition that it is possible to produce biosynthetic hGH which has attached to it a specific negatively charged pre-sequence which can be cleaved enzymatically by means of a particular dipeptidyl aminopeptidase in a high yield, and which gives products by the enzymatic cleavage which can be separated satisfactorily by known purification methods, such as chromatography. Thus, it is possible to obtain homogeneous authentic hGH in commercial amounts and suitable for therapeutic treatment.

In the process of the present invention, which is characterized by the features defined in the characterizing portion of claim 1, the enzyme Dipeptidyl aminopeptidase (DAP I) (E.C. 3.4.14.1) is used for cleaving a specific amino terminal extension on hGH, the extension having at least one amino acid selected from Asp and Glu. This enzyme is specific in that it can cleave dipeptides from the N-terminus of peptides and proteins, i.a. on the following conditions:

a) that the N-terminal amino acid in the protein/peptide is not Lys or Arg.

b) that the second or the third amino acid from the N-terminus of the protein/peptide is not Pro.

The N-terminal sequence of hGh is

$Phe^1Pro^2Thr$-Ile

and this means that DAP I cannot cleave the bonds in hGH indicated by 1 and 2.

It is clear that since DAP I cleaves dipeptides, the extension must consist of an even number of amino acids in order for the amino terminal extended hGH to be converted to hGH without an additional amino acid.

Since the enzyme has the above-mentioned specificity, it is clear that the extension must not contain Lys or Arg at the uneven sites, and if Pro occurs, it may only be positioned as an N-terminal amino acid since, otherwise, the cleavage of dipeptides would stop before the entire extension has been cleaved.

Since no enzyme reactions can be expected to lead to a 100% conversion, it is necessary to introduce amino acids in the extension which enable analytic and preparative separation of authentic hGH from amino terminal extended hGH. In principle, This may be done by selecting amino acids with charged side chains, i.e. Glu or Asp (negatively charged side chains) or Arg, Lys or His (positively charged side chains). Then, by anion exchange and cation exchange (analytic or preparative) or by anionic and cationic electrophoresis it will be possible to separate amino terminal extended hGH from (authentic) hGH-provided that no combination of positively and negatively amino acids with charged side chains in the extension has been selected which neutralize each other in terms of charge.

According to the invention, at least one of the amino acids contained in the dipeptide component, which is attached directly to the N-terminus of hGH, is selected from Asp and Glu because it may then be observed whether the entire amino terminal extension has been cleaved. However, it is most expedient that the last amino acid residue in the extension is Asp or Glu. This is particularly important when the microorganism in vivo only partly cleaves the N-terminal methionine residue.

It is most expedient that an amino acid with charged side chains in the amino terminal extension to hGH is exclusively negatively charged. This prevents amino terminal extended hGH, partly enzymatically converted amino terminal extended hGH and authentic hGH from having the same net charge at any time.

In hGH, slight deamidation of certain Gln and Asn residues takes place, i.e. Gln and Asn are converted to Glu and Asp, respectively-i.e. amino acids with negatively charged side chains. The specified composition of the dipeptide component which is attached directly to the N-terminal of hGH avoids the situation of one or more deamidations in hGH neutralizing the positive charge/charges present in the extension. Such neutralization of charges would make it impossible to separate possibly unreacted deamidated amino terminal extended hGH by ion exchange from the enzymatically formed hGH.

Examples of particularly suitable amino terminal extensions which may be cleaved with DAP I are

1. Met-Glu-Ala-Glu
2. $(Ala\text{-}Glu)_r$, wherein r is an integer from 1 to 12
3. Met-Phe-Glu-Glu
4. Thr-Glu-Ala-Glu
5. Met-Asp-Ala-Asp
6. Met-Glu-Ala-Asp

These and other suitable amino terminal extensions may be obtained by fermenting in a suitable substrate a microorganism transformed with a plasmid, which codes for human growth hormone with these attached amino terminal extensions.

In some specific pre-sequences, methionine, which is the N-terminal amino acid in all proteins formed in E.coli, is cleaved enzymatically in the microorganism after expression of the protein. This results e.g. in the above-mentioned amino terminal extended hGH proteins.

These proteins are purified by conventional purification methods. The amino terminal extension is cleaved selectively and in a high yield, and the formed hGH may then easily be separated from any residues of partly converted amino terminal extended hGH by anion exchange.

The process of the invention will be illustrated more fully below by means of some working examples.

Example 1

Preparation of active enzyme or enzyme complex

A leucine aminopeptidase preparation from Sigma was used as the starting material for the production of active enzyme or enzyme complex, the active enzyme being fractionated together with leucine aminopeptidase (LAP). The active enzyme may be fractionated from LAP by IE-HPLC (ion exchange HPLC) on a highly soluble Mono Q™ from Pharmacia.

Fractionation

Column: 0.196 $cm^2 \times 5$ cm packed with Mono Q™ (Pharmacia), 10 $\mu m \pm 0.2$ $\mu m$.
Buffer A: 20 mM Tris-Cl, 5 mM $MgCl_2$ pH = 8.5
Buffer B: 20 mM Tris-Cl, 5 mM $MgCl_2$, 500 mM NaCl pH = 8.5.
Flow rate: 1 ml/min.
Injection volume: 500 $\mu l$.
Detection: 280 nm, 0.5 AUFS.
Temperature: 20 °C.

| Gradient Table: | | | |
|---|---|---|---|
| Time | %A | %B | Gradient form |
| 0 | 100 | 0 | - |
| 2 | 100 | 0 | linear |
| 18 | 0 | 100 | linear |

Fractions: 1 $min^{-1}$.

The used leucine aminopeptidase preparation from Sigma, L-1503, lot No. 14F-8155 containing 10 mg of LAP/ml and 120 U/mg, is diluted to 2.6 mg/ml with buffer A. 500 µl were applied to the column. The UV profile of the fractionation appears from Figure 1, which shows that the preparation is inhomogeneous. 17 fractions are collected and checked for leucine aminopeptidase activity and the specific activity for conversion of Ala-Glu-hGH to hGH.

The leucine aminopeptidase activity is measured spectrophotometrically by transforming L-leucine amide to L-leucine and ammonia at 238 nm, pH = 8.5, as described in (ref. 1) E. Merck: Analytisches Zentrallab. Prüfungsvorschrift code No. AZL 0526675. 300 µl of each fraction are pre-incubated at 40 °C for 2 hours prior to the activity measurement The result of the activity measurement appears from the drawing.

The specific activity for the conversion of Ala-Glu-hGH to hGH is measured in the following manner: 100 µl of the individual fractions are admixed with 100 µl of Ala-Glu-hGH (1 mg/ml in 5 mM $MgCl_2$ 10 mM Tris pH = 8.5), produced as described in Example 2, pH is adjusted to 4.2. The reaction mixture is analyzed after 2 hours by polyacrylamide gel electrophoresis on a 10% gel. The degree of conversion is determined by laser scanning of the gels. The result appears from the drawing.

The leucine aminopeptidase activity in the individual fractions is indicated in the drawing. There is only LAP activity in fractions 13 and 14. A balance calculation on LAP activity shows that all applied LAP activity is eluted in these two fractions.

Activity for the conversion of Ala-Glu-hGH to hGH is present in the fractions 9-15 with maximum in fraction 12, and then the activity decreases rapidly. Thus, it is clear that two enzyme activities are present, and that it is possible by the given fractionation method to separate these. It is demonstrated that the specific activity for the conversion of Al-Glu-hGH is due to the presence of the enzyme DAP I.

Preparation of Ala-Glu-hGH by biosynthesis.

A cloned DNA sequence which codes for a protein having an amino acid sequence like human growth hormone, hGH (191 amino acid residues, the first four amino acids of which are Phe-Pro-Thr-Ile) is coupled with the following synthetically produced, dual-stranded DNA sequence so that the 3'end of the + strand is coupled to the +5' end of the above-mentioned gene, and the 5' end of the synthetic DNA sequence strand is coupled to the 3' end of the above-mentioned gene by blunt end ligature

+5′ CGATG GCT GAA

−3′ TAC CGA CTT

where the 2 first nucleotides in the + strand are a ClaI restriction site overhang, and the following nucleotide sequences code for the amino acids Met-Ala-Glu-.

The above-mentioned gene is introduced by ordinary gene cloning techniques into an expression plasmid containing a fusioned Trp-Lac promoter as well as the SD sequence AGGA. This structure should express Met-Ala-Glu-hGH.

This plasmid structure is then introduced in an E. coli cell by prior art techniques. A suitable clone containing the above-mentioned structure is isolated and cultivated in a 5 l scale. The cells were harvested by centrifugation and are suspended in a small volume and lyzated using a so-called "French press".

The expected fusion protein could be demonstrated in the above-mentioned bacterial extract by immunological methods using hGH antibodies, corresponding to a concentration of 200 mg/l in the culture medium. The fusion protein is purified conventionally by anion exchange, ammonium sulfate precipitation and hydrophobic chromatography.

The purified (expected) Met-Ala-Glu-hGH was evaluated to be more than 99% pure, evaluated by SDS electrophoresis.

An amino terminal sequence determination showed that the purified hGH material had the sequence Ala-Glu-hGH, which means that Met has been cleaved by an E. coli enzyme.

Enzymatic cleavage of Ala-Glu-hGH to hGH

100 mg of Ala-Glu-hGH in 20 mM Tris-Cl, pH 4.2 (1.5 mg/ml) were admixed with 7 mg of enzyme fraction with DAP I activity, isolated from pork kidneys as stated above.

The reaction mixture was then incubated at 40 °C. After 4-1/2 hours the mixture was cooled to 4 °C. The cooled reaction mixture was then fractionated by anion exchange, and following this the mean peak (hGH product) was isolated. The yield was 90%.

The hGH product was evaluated to be more than 99% pure, evaluated by SDS electrophoresis. An amino terminal determination (Edman degradation) showed that the amino terminal sequence of the hGH product was Phe-Pho-Thr-Ile-Pro-, i.e. as for authentic hGH.

The biological activity of the hGH product was determined by a tibia test and was found to be 2.5 IU/mg, which is also the case with authentic hGH.

Example 2

Preparation of hGH from Met-Glu-Ala-Glu-hGH with dipeptidyl aminopeptidase I

Met-Glu-Ala-Glu-hGH is produced by gene techniques in principle as described in Example 1. Met-Glu-Ala-Glu-hGH is purified from the fermentation product by anion exchange and hydrophobic interaction chromatography.

The purified Met-Glu-Ala-Glu-hGH was evaluated to be more than 99% pure by IE-HPLC and SDS electrophoresis.

An amino terminal sequence determination showed that the purified hGH had the sequence Met-Glu-Ala-Glu-Phe-Pro-Thr-Ile-Pro-Leu, where the last six amino acids correspond to the N-terminus in hGH. 200 ml of Met-Glu-Ala-Glu-hGH (2.0 mg/ml) in 20 mM Tris, 10 mM citric $acid_1$ 25 mM NaCl, pH 4.2 were admixed with 10,000 mU (corresponding to 3.3 mg) dipeptidyl aminopeptidase I (E.C. 3.4.14.1) from Boehringer Mannheim. Other makes may be used as well. The pH value is optionally readjusted to 4.2.

The reaction mixture was then incubated at 40 °C for 60 minutes, resulting in a more than 98% conversion of Met-Glu-Ala-Glu-hGH to hGH. The reaction mixture was cooled to 40 °C after completed reaction. The further purification comprises isoprecipitation, gel filtration and an anion exchange.

The hGH product was evaluated to be more than 99% pure evaluated by IE-HPLC and SDS electrophoresis. An amino terminal sequence determination by Edman degradation showed that the amino terminal sequence of the hGH product was Phe-Pro-Thr-Ile-Pro-Leu, i.e. as for authentic hGH.

The biological activity of the hGH product was determined by a tibia test and was found to be equipotent with pituitary hGH.

Example 3

Preparation of hGH from Ala-Glu-hGH with dipeptidyl aminopeptidase I

Met-Ala-Glu-hGH is produced by gene techniques in principle as described in Example 1. Met is cleaved in vivo so that the protein formed by the fermentation is Ala-Glu-hGH. This is purified conventionally by anion exchange and hydrophobic interaction chromatography.

The purified Ala-Glu-hGH was evaluated to be more than 99% pure by IE-HPLC and SDS electrophoresis.

An amino terminal sequence analysis showed that the purified hGH product had the sequence Ala-Glu-Phe-Pro-Thr-Ile-Leu-Pro-Leu, where the last six amino acids correspond to the N-terminus in hGH.

100 ml of Ala-Glu-hGH (2.2 mg/ml) in 20 mM Tris, 10 mM citric acid, 25 mM NaCl, pH 4.2 were admixed with 8800 mU (corresponding to 2.9 mg) dipeptidyl aminopeptidase I (E.C. 3.4.14.1) from Boehringer Mannheim. Other makes may be used as well. The pH value is optionally readjusted to 4.2.

The reaction mixture was incubated at 40 °C for 60 minutes, resulting in a more than 98% conversion of Ala-Glu-hGH. The reaction mixture was cooled to 4 °C after completed reaction. The further purification comprises isoprecipitation, gel filtration and an anion exchange.

The hGH product was found to be more than 99% pure evaluated by IE-HPLC and SDS electrophoresis. An amino sequence determination by Edman degradation showed that the amino terminal sequence of hGH was Phe-Pro-Thr-Ile-Pro-Leu, i.e. as for authentic hGH.

The biological activity of the hGH product was determined by a tibia test and was found to be equipotent with pituitary hGH.

Example 4

Preparation of hGH from Met-Phe-Glu-Glu-hGH with dipeptidyl aminopeptidase I

Met-Phe-Glu-Glu-hGH is produced by gene techniques in principle as described in Example 1. Met-Phe-Glu-Glu-hGH is purified from the fermentation product by anion exchange and hydrophobic interaction chromatography.

The purified Met-Phe-Glu-Glu-hGH was evaluated to be more than 99% pure by IE-HPLC and SDS electrophoresis.

An amino terminal sequence determination showed that the purified hGH had the sequence Met-Phe-Glu-Glu-Phe-Pro-Thr-Ile-Pro-Leu, where the last six amino acids correspond to the N-terminus in hGH.

100 ml of Met-Phe-Glu-Glu-hGH (1.5 mg/ml) in 20 mM Tris, 10 mM citric acid, 25 mM NaCl, 1 mM L-Cysteine pH 4.2 were admixed with 15,000 mU (corresponding to 5.0 mg) dipeptidyl aminopeptidase I (E.C. 3.4.14.1) from Boehringer Mannheim. Other makes may be used as well. The pH value is optionally readjusted to 4.2.

The reaction mixture was then incubated at 40°C for 60 minutes, resulting in a more than 98% conversion of Met-Phe-Glu-Glu-hGH to hGH. The reaction mixture is cooled to 4°C after completed reaction. The further purification comprises isoprecipitation, gel filtration and an anion exchange.

The hGH product was evaluated to be more than 99% pure evaluated by IE-HPLC and SDS electrophoresis. An amino terminal sequence determination by Edman degradation showed that the amino terminal sequence of the hGH product was Phe-Pro-Thr-Ile-Pro-Leu, i.e. as for authentic hGH.

The biological activity of the hGH product was determined by a tibia test and was found to be equipotent with pituitary hGH.

Example 5

Preparation of hGH from Ala-Glu-Ala-Glu-hGH with dipeptidyl aminopeptidase I

Met-Ala-Glu-Ala-Glu-hGH is produced by gene techniques in principle as described in Example 1. Met is cleaved in vivo so that the protein formed by the fermentation is Ala-Glu-Ala-Glu-hGH. This is purified conventionally by anion exchange and hydrophobic interaction chromatography.

The purified Ala-Glu-Ala-Glu-hGH was evaluated to be more than 99% pure by IE-HPLC and SDS electrophoresis.

An amino terminal sequence analysis showed that the purified hGH product had the sequence Ala-Glu-Ala-Glu-Phe-Pro-Thr-Ile-Pro-Leu, where the last six amino acids correspond to the N-terminus in hGH.

100 ml of Ala-Glu-Ala-Glu-hGH (2.0 mg/ml) in 20 mM Tris, 10 mM citric acid, 25 mM NaCl, pH 4.2 were admixed with 2000 mU (corresponding to 6.7 mg) Dipeptidyl Aminopeptidase I (E.C. 3.4.14.1) from Boehringer Mannheim. Other makes may be used as well. The pH value is optionally readjusted to 4.2.

The reaction mixture was incubated at 40°C for 60 minutes, resulting in a more than 98% conversion of Ala-Glu-Ala-Glu-hGH to hGH. The reaction mixture was cooled to 4°C after completed reaction. The further purification comprises isoprecipitation, gel filtration and an anion exchange.

The hGH product was found to be more than 99% pure evaluated by IE-HPLC and SDS electrophoresis. An amino terminal sequence determination by Edman degradation showed that the amino terminal sequence of hGH was Phe-Pro-Thr-Ile-Pro-Leu, i.e. as for authentic hGH.

The biological activity of the hGH product was determined by a tibia test and was found to be equipotent with pituitary hGH.

**Claims**

**1.** A process for producing authentic human growth hormone (hGH), wherein a biosynthetically formed amino terminal extended hGH is digested with an aminopeptidase, CHARACTERIZED IN that the amino terminal extended hGH used has the formula X-hGH, wherein X is an amino acid sequence having an

even number of amino acids, which do not neutralize each other in terms of charge, the first one of which, as seen from the N-terminus being different from Lys and Arg, all other uneven amino acids being different from Pro, Lys, and Arg and all even amino acids being different from Pro, and that the dipeptide component, which is attached directly to the N-terminus of hGH comprises at least one amino acid selected from Asp and Glu, the other being an amino acid which is not positively charged, and that the aminopeptidase used is dipeptidyl aminopeptidase I (E.C. 3.4.14.1).

**2.** A process according to claim 1, CHARACTERIZED IN that the last amino acid in the amino acid sequence X is Asp or Glu.

**3.** A process according to claim 1, CHARACTERIZED IN that the penultimate amino acid in the amino acid sequence X is Asp or Glu.

**4.** A process according to claim 1, CHARACTERIZED IN that both the last and the penultimate amino acid in the amino acid sequence X is Asp or Glu.

**Patentansprüche**

**1.** Verfahren zur Herstellung von authentischem Human-Wachstumshormon (hGH), worin ein biosynthetisch gebildetes, amino-terminal verlängertes hGH mit einer Aminopeptidase verdaut wird, DADURCH GEKENNZEICHNET, daß das verwendete amino-terminal verlängerte hGH die Formel X-hGH hat, in der X eine Aminosäure-Sequenz mit einer geraden Zahl von Aminosäuren ist, die einander ladungsmäßig nicht neutralisieren, von denen die erste, vom N-terminalen Ende gesehen, von Lys und Arg verschieden ist, alle anderen ungeradzahligen Aminosäuren von Pro, Lys und Arg verschieden sind und alle geradzahligen Aminosäuren von Pro verschieden sind, und daß die Dipeptid-Komponente, die direkt in der N-terminalen Position an hGH gebunden ist, wenigstens eine aus Asp und Glu ausgewählte Aminosäure umfaßt, wobei die andere eine Aminosäure ist, die nicht positiv geladen ist, und daß die verwendete Aminopeptidase Dipeptidylaminopeptidase I (E.C. 3.4.14.1) ist.

**2.** Verfahren nach Anspruch 1, DADURCH GEKENNZEICHNET, daß die letze Aminosäure in der Aminosäure-Sequenz X Asp oder Glu ist.

**3.** Verfahren nach Anspruch 1, DADURCH GEKENNZEICHNET, daß die vorletze Aminosäure in der Aminosäure-Sequenz X Asp oder Glu ist.

**4.** Verfahren nach Anspruch 1, DADURCH GEKENNZEICHNET, daß sowohl die letzte als auch die vorletze Aminosäure in der Aminosäure-Sequenz X Asp oder Glu sind.

**Revendications**

**1.** Procédé de production d'hormone de croissance humaine (hGH) authentique, dans lequel une hGH à extension amino-terminale formée par biosynthèse est digérée avec une aminopeptidase, caractérisé en ce que la hGH à extension amino-terminale utilisée répond à la formule X-hGH, dans laquelle X représente une séquence d'amino-acides ayant un nombre pair d'amino-acides, qui ne se neutralisent pas mutuellement en termes de charge, dont le premier, en effectuant l'observation par l'extrémité N-terminale, est différent de Lys et Arg, tous les autres amino-acides impairs étant différents de Pro, Lys et Arg et tous les amino-acides pairs étant différents de Pro, et en ce que le constituant dipeptidique, qui est fixé directement à l'extrémité N-terminale de la hGH, comprend au moins un amino-acide choisi entre Asp et Glu, l'autre amino-acide étant un amino-acide qui n'est pas chargé positivement, et en ce que l'aminopeptidase utilisée est la dipeptidyl-aminopeptidase I (E.C. 3.4.14.1).

**2.** Procédé suivant la revendication 1, caractérisé en ce que le dernier amino-acide dans la séquence d'amino-acides X est Asp ou Glu.

**3.** Procédé suivant la revendication 1, caractérisé en ce que l'avant-dernier amino-acide dans la séquence d'amino-acides X est Asp ou Glu.

4. Procédé suivant la revendication 1, caractérisé en ce que le dernier amino-acide et l'avant-dernier amino-acide dans la séquence d'amino-acides X consistent en Asp ou Glu.

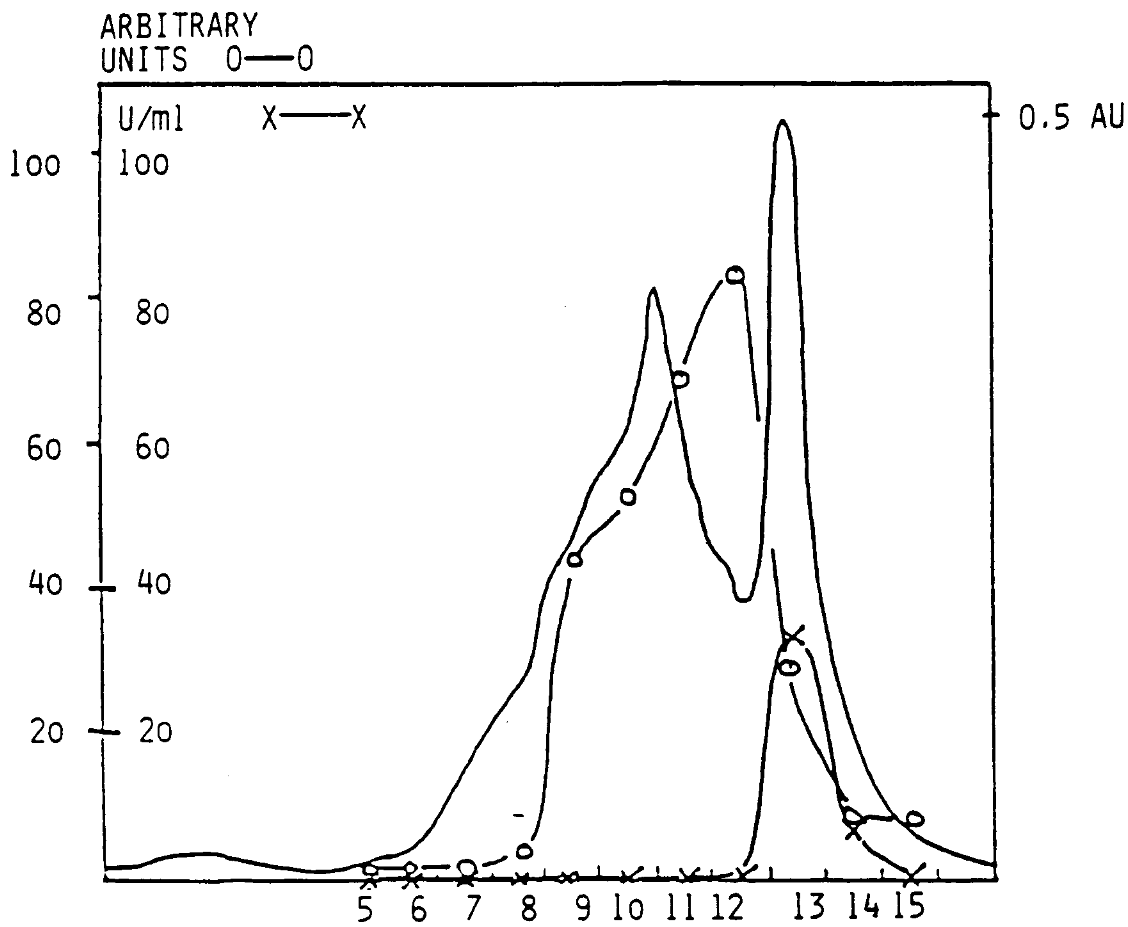


— A-280 NM (PROTEIN)

O—O ENZYME ACTIVITY CAPABLE OF CONVERTING AE-HGH TO HGH AT pH=4.2

X—X LEUCINE AMINOPEPTIDASE ACTIVITY